# EUROPÄISCHE PATENTANMELDUNG

(11) **EP 1 273 913 A1**
(43) Veröffentlichungstag der Anmeldung: **08.01.2003**
(21) Anmeldenummer: 01401756.0
(22) Anmeldetag: 02.07.2001
(51) Int. Cl.: G01N 33/50, C12M 1/12, C12M 3/06, C12M 1/22

(54) **Verfahren zur Bestimmung der Interaktionen zwischen Keratinocyten und Neuronen**

(71) Anmelder: Cognis France S.A., 31360 Saint-Martory (FR)
(72) Erfinder: PAULY, Gilles, 54000 Nancy (FR); CONTET-AUDONNEAU, Jean-Luc, 54130 Saint-Max (FR); DANOUX, Louis, 54420 Saulxures-Les-Nancy (FR); ULMANN, Lauriane, 67000 Strasbourg (FR); JOVER, Emmanuel, 67000 Strasbourg (FR); SCHLICHTER, Rémy, 67240 Oberhoffen sur Moder (FR)
(74) Vertreter: Cabinet HERRBURGER

(57) **Zusammenfassung**

Vorgeschlagen wird ein Verfahren zur Bestimmung von Interaktionen zwischen Keratinocyten und Neuronen aus Spinalganglien bei dem die beiden Zelltypen in Co-Kultur in zwei separaten Kompartimenten kultiviert werden, welche den Durchlass und den Austausch von Nervenfasern und/oder löslichen Faktoren zulassen, sowie eine Vorrichtung zur Bestimmung der Interaktionen zwischen diesen beiden Zelltypen und die Verwendung dieser beiden Zelltypen zur Untersuchung von unterschiedlichen Effekten auf die Interaktion zwischon diesen beiden Zelltypen.

## Beschreibung

### Gebiet der Erfindung

Die Erfindung liegt auf dem Gebiet der kosmetischen und/oder dermatologischen Untersuchungsmethoden und betrifft ein Verfahren zur Bestimmung der Interaktionen zwischen Keratinocyten und Neuronen aus Spinalganglien sowie eine Vorrichtung zur Bestimmung der Interaktionen zwischen diesen beiden Zelltypen und die Verwendung dieser beiden Zelltypen zur Untersuchung von unterschiedlichen Effekten auf die Interaktion zwischon diesen beiden Zelltypen.

### Stand der Technik

Die Haut als das größte Organ des Menschen übt zahlreiche lebenswichtige Prozesse aus. Neben ihren Schutzfunktionen gegenüber Umwelteinflüssen ist die Haut an verschiedenen Stoffwechsel-, Speicherungs- und Regulationsvorgängen des Körpers beteiligt. Sie ist mit dem übrigen Körper durch den Blutkreislauf sowie durch das Lymph- und Nervensystem eng verbunden. Sie ist aus mehreren Schichten aufgebaut und mit Blutgefäßen, Nerven und Sinnesorganen ausgestattet.

Als äußerste Schicht bildet die Epidermis (Oberhaut) die eigentliche Schutzschicht gegenüber der Umwelt. In der Epidermis der Haut finden sich neben anderen Zelltypen hauptsächlich Keratinocyten. Diese Zellen spielen eine entscheidende Rolle beim Wachstum und der Differenzierung der Zellen. Sie bilden das Keratin, welches zum Aufbau der Hornschicht der Haut verwendet wird.

Die menschliche Haut ist mit zahlreichen Nerven ausgestattet. Man unterscheidet zwei Arten von Nerven, die autonomen oder auch vegetativen Nerven und die sensorischen oder auch zerebrospinalen Nerven. Die sensorischen Nerven leiten die äußeren und die inneren Hautreize an die Epidermis weiter. Die Zellkörper dieser Nervenzellen, die sogenannten Neuronen sind als Ansammlungen ausserhalb des Zentralnervensystems in sogenannten Ganglien zusammengeschlossen. Diese Ganglien werden als Spinalganglien oder als DRG (dorsal root ganglia) bezeichnet und stammen aus dorsalen sensiblen Nervenwurzeln. Neben diesem Zellkörper besitzen die Neuronen mehrere Zellfortsätze; die langen Nervenfasern werden als Neurite bezeichnet, die kurzen, stark verästelten Fasern nennt man Dendrite. Über Synapsen werden die Neuronen mit anderen Zellen verbunden.

Unter den vielen Arten von Nervenzellen, welche diese Ganglien formen gibt es die vom Typ Aδ und vom Typ C, welche sich in periphere Richtung ausweiten und von denen die freien Nervenenden den nervus plexus bilden. Dieser nervus plexus liegt direkt oberhalb der Dermis und penetriert in die Epidermis hinein. Der sub-epidermale nervus-plexus ist verantwortlich für die Erkennung der Stimulierung von Nozizeptoren.

Nozizeptoren (Nozisensoren) sind Nervenzellen in der Haut, die der Aufnahme von gewebsschädigenden oder potentiell gewebsschädigenden Reizen dienen. Es handelt sich dabei um freie Nervenendigungen, d. h. die peripheren Fortsätze dieser Neurone, deren Zellkörper in Ganglien entlang der Wirbelsäule (Spinalganglien) liegen, enden ohne spezielle Endorgane im Gewebe. Nozizeptoren sind gegenüber mechanischen, thermischen und chemischen Reizen empfindlich. Diese Reize werden in elektronische Impulse umgewandelt (Transduktion) und zum Rückenmark weitergeleitet. Bei den Fortsätzen der Nozizeptoren handelt es sich um sehr dünne Nervenfasern mit relativ niedriger Leitungsgeschwindigkeit.

Keratinocyten sind in der Lage eine Vielzahl von Substanzen zu produzieren. Darunter finden sich sowohl Cytokine, wie beispielsweise ein Protein, welches als Nerven-Wachstumsfaktor NGF (nerv growth factor) bezeichnet wird, als auch Moleküle mit autokrinen und parakrinen Effekten. Unter normalen Bedingungen werden diese Substanzen nicht produziert und damit auch nicht sekretiert. Durch den Einfluß äußerer Reize und Stimulierungen werden die Zellen jedoch dazu angeregt diese Substanzen zu produzieren, die dann als Signalsubstanzen an der Signalübertragung beteiligt sind. Cytokine und Chemokine regulieren die Proliferation und die Differentiation elementarer Hautbestandteile und werden als Immunantwort und bei Entzündungen produziert, wenn die Stimulierung der Produktion durch eine Wunde oder Läsion hervorgerufen wurde.

Der Metabolismus der Keratinocyten kann durch Messenger Moleküle oder Neurotransmitter der Nervenenden des nervus plexus beeinflußt werden (vgl: *Huang IT, Lin WM, Shun CT, Hsieh ST, Influence of cutaneous nerves on keratinocyte proliferation and epidermal thickness in mice. Neuroscience, 94: 965-973, 1999).* Hier zeigt sich, dass die gegenseitige Beeinflussung zwischen Keratinocyten und sensorischen Neuronen bereits in einem frühen Stadium der kutanen Entwicklung einsetzt.

Studien an der Haut und an der sensorischen Innervierung der Haut durch Nervenzellen liefern einen wichtigen Beitrag zur Physiologie der Nervenzellen und für kosmetische und pharmazeutische Untersuchungen. Die zum Stand der Technik zählenden *in vivo* Studien zur Interaktion zwischen sensorischen Nervenzellen und Keratinocyten nutzen Abschnitte von Nerven ohne den Zellkörper und untersuchen den Einfluss auf Keratinocyten. Die Abschnitte der Nervenenden werden dabei in eine neue Umgebung gebracht was zu einer Verfälschung der eigentlichen Interaktionen führt. Die natürlichen Bedingungen sind durch diese *in vivo* Studien einerseits aufgrund der wichtigen Distanz zwischen den Keratinocyten in der Epidermis und den Neuronen und andererseits aufgrund der entscheidenden Anzahl an Molekülen die zur Innervierung der Interaktion benötigt werden, sehr schwierig nachzustellen. Diese Art der Studien sind für eine verlässliche Aussage zur Interaktion zwischen Neuronen und Keratinocyten nicht geeignet. Sie lassen auch keinen Schluss zu über die unterschiedlichen Effekte auf die Interaktion und auf die Bildung von Hautzellen, die durch verschiedene Faktoren wie Umwelteinflüsse oder Strassfaktoren ausgelöst werden.

Einige vereinfachte Studien zur Interaktion von Keratinocyten und Neuronen wurden *in vitro* bereits durchgeführt um die Signalübertragung der Nervenenden in der Epidermis zu verstehen (vgl.: 1. *Reeh PW*, *Sensory receptors in mammalian skin in an in vitro preparation, Neurosci. Lett., 66: 141-146, 1986 -* 2. *Reeh PW, Sensory receptors in a mammalian skin-nerve in vitro preparation, Prog. Brain Res.*, *74: 271-276).* In diesen Studien wurden jedoch keine kompletten Neuronen mit Zellkörper und Nervenenden untersucht. In anderen *in vitro* Untersuchungen zur Differenzierung von sensorischen Neuronen wurde der Einfluss auf die Bildung von Geweben untersucht, indem die Neuronen auf einer Mono-Schicht der betreffenden Gewebe kultiviert wurden (vgl.: *Hall AK, Ai X, Hickman GE, MacPhedran SE, Nduaguba CO, Robertson CP, The generation of neuronal heterogeneity in a rat sensory ganglion, J. Neurosci, 17: 2775-2784, 1997).*

Eine *in vitro* Untersuchung, bei der Keratinocyten und sensorische Neuronen gemeinsam kultiviert wurden, wird beschrieben von Steinschneider et al. in *Steinschneider R*, *Quelven E, Grousson J, Laurent JC, Screening of molecules with anti-inflammatory properties and for sensitive skin applications using rat sensory neurons cultured or not with human keratinocytes, Cosm'ing, 16-18 May, Saint-Malo Franc,* 200. Der Nachteil dieser *in vitro* Untersuchnung ist jedoch, dass die Keratinocyten und Neuronen nebeneinander gemeinsam kultiviert werden und in einem direkten Kontakt stehen. Die natürlich vorkommende räumliche Trennung der Zellen, die über Nervenfasern und Synapsen überbrückt wird, kann durch dieses Modell nicht nachgestellt werden, sodass die erhaltenen Ergebnisse der eigentlichen Interaktionen zwischen diesen Zelltypen nicht korrekt wiedergegeben werden können. Desweiteren werden Zelltypen unterschiedlicher Herkunft aus unterschiedlichen Organismen nebeneinander kultiviert, was ebenfalls zu einer Verfälschung natürlicher Bedingungen führt.

Die komplexe Aufgabe der Erfindung hat somit darin bestanden, ein Verfahren zur Verfügung zu stellen, mit dem es möglich wird, *in vitro* Untersuchungen durchzuführen, die ein möglichst realitätsnahes Modell zur Analyse der Interaktion zwischen Neuronen und Keratinocyten liefern. Es sollte möglich werden mit diesem Verfahren die schnelle und die langsame Interaktion dieser beiden Zelltypen zu untersuchen.

Eine weitere Aufgabe der Erfindung hat darin bestanden, ein Verfahren zur Verfügung zu stellen, bei dem ein Modell verwendet wird, welches sowohl die Kultivierung tierischer Zellen als auch die Kultivierung menschlicher Zelllinien erlaubt.

Desweiteren sollte das erfindungsgemäße Verfahren die Untersuchung pathogener Mediatoren sowie die Identifizierung und Optimierung neuer Wirkstoffe und Methoden für die Kosmetik und für die Behandlung kutaner Krankheiten ermöglichen.

Weiterhin sollte es möglich werden, unterschiedliche Interaktionen zu untersuchen, die ausgelöst werden durch Effekte der unterschiedlichsten Faktoren wie Stressfaktoren oder ähnliches. Besonders sollte geklärt werden können, welche Interaktionen durch diese Effekte auf die Bildung der Hornschicht der Haut oder auf die Sekretion unterschiedlicher Moleküle, die von Keratinocyten als Reaktion auf Stimulierung synthetisiert werden, ausgelöst werden.

### Beschreibung der Erfindung

Gegenstand der Erfindung ist ein Verfahren zur Bestimmung von Interaktionen zwischen Keratinocyten und Neuronen aus Spinalganglien bei dem die beiden Zelltypen in Co-Kultur in zwei separaten Kompartimenten kultiviert werden, welche den Durchlass und den Austausch von Nervenfasern und löslichen Faktoren zulassen.

Der erfindungsgemäß verwendete Begriff "Keratinocyten" bezeichnet Zellen aus der Epidermis, die auch als Epithelzellen bezeichnet werden. Keratinocyten bilden das Keratin, welches zum Aufbau der Hornschicht der Haut verwendet wird und sind weiterhin in der Lage eine Vielzahl von Substanzen zu produzieren. Darunter finden sich sowohl Cytokine, wie beispielsweise der Wachstumsfaktor NGF (nerv growth factor) als auch Moleküle mit autokrinen und parakrinen Effekten. Unter normalen Bedingungen werden diese Substanzen nicht produziert und damit auch nicht sekretiert. Durch den Einfluß äußerer Reize und Stimulierungen werden die Zellen jedoch dazu angeregt diese Substanzen zu produzieren, die dann als Signalsubstanzen an der Signalübertragung beteiligt sind. Cytokine und Chemokine regulieren die Proliferation und die Differentiation elementarer Hautbestandteile und werden als Immunantwort und bei Entzündungen produziert, wenn die Stimulierung der Produktion durch eine Wunde oder Läsion hervorgerufen wurde.

Unter Neuronen sind im Sinne der Erfindung Nervenzellen als Ganzes zu verstehen. Die Begriffe Neuron und Nervenzelle werden synonym verwendet. Diese setzen sich zusammen aus einem Zellkern oder Zellkörper und mehreren Zellfortsätzen, den sogenannten Nervenfasern. Neuronen im Sinne der Erfindung sind die sensorischen oder auch zerebrospinalen Nerven. Die sensorischen Nerven leiten die äußeren und die inneren Hautreize an die Epidermis weiter. Die Zellkörper der Neuronen oder Nervenzellen sind als Ansammlungen ausserhalb des Zentralnervensystems in sogenannten Ganglien zusammensgeschlossen. Diese Ganglien werden als Spinalganglien oder als DRG (dorsal root ganglia) bezeichnet und stammen aus dorsalen sensiblen Nervenwurzeln. Neben diesem Zellkörper besitzen die Neuronen mehrere Zellfortsätze; die langen Nervenfasern werden als Neurite bezeichnet, die kurzen, stark verästelten Fasern nennt man Dendrite. Natürlicherweise werden die Neuronen über Synapsen mit anderen Zellen verbunden.

Unter den vielen Arten von Nervenzellen, welche diese Ganglien formen gibt es die vom Typ Aδ und vom Typ C, welche sich in periphere Richtung ausweiten und von denen die freien Nervenenden den nervus plexus bilden. Dieser nervus plexus liegt direkt oberhalb der Dermis und penetriert in die Epidermis hinein. Der sub-epidermale nervus-plexus ist verantwortlich für die Erkennung der Stimulierung von Nozizeptoren.

Nozizeptoren (Nozisensoren) sind Nervenzellen in der Haut, die der Aufnahme von gewebsschädigenden oder potentiell gewebsschädigenden Reizen dienen. Es handelt sich dabei um freie Nervenendigungen, d. h. die peripheren Fortsätze dieser Neurone, deren Zellkörper in Ganglien entlang der Wirbelsäule (Spinalganglien) liegen, enden ohne spezielle Endorgane im Gewebe. Nozizeptoren sind gegenüber mechanischen, thermischen und chemischen Reizen empfindlich. Diese Reize werden in elektronische Impulse umgewandelt (Transduktion) und zum Rückenmark weitergeleitet. Bei den Fortsätzen der Nozizeptoren handelt es sich um sehr dünne Nervenfasern mit relativ niedriger Leitungsgeschwindigkeit.

Unter dem Begriff "Co-Kultur" ist im Sinne der Erfindung eine Möglichkeit zu verstehen, mit der die beiden Zelltypen Neuronen und Keratinocyten zwar räumlich getrennt in zwei Kompartimenten also zwei physikalisch voneinander getrennten Teilbereichen kultiviert werden, jedoch ein Austausch und ein Durchlass von löslichen Faktoren und Nervenfasern möglich ist. Der Austausch von Keratinocyten und ganzen Nervenzellen wird verhindert. Die Zellen können in dem gleichen Medium unter gleichen Bedingungen kultiviert werden.

Die mögliche Kultivierung in zwei Kompartimenten kann erfindungsgemäß errreicht werden durch eine Vorrichtung, bei der durch eine Trennscheibe oder Trennwand ein Gefäß in zwei Kompartimente geteilt wird. Bevorzugt wird die Trennscheibe oder Trennwand mit einer Mischung aus Cellulose und Schmiermitteln an das Gefäß angehaftet. Bei dem Gefäß handelt es sich bevorzugt um eine Petrischale. Bei der Trennscheibe oder Trennwand kann es sich um eine eine Membran, Platte, Scheibe, Wand oder einen Ring handeln, bevorzugt handelt es sich um einen Ring. Das Material dieser Trennscheibe oder der Trennwand ist im Prinzip frei wählbar zwischen Materialien, die sich durch die Kultivierungsbedingungen der beiden Zelltypen und/oder dem Haftungsmaterial nicht auflösen und nicht zerstört werden. Bevorzugt verwendet man als Material für die Trennscheibe oder Trennwand Glas.

In einer bevorzugten Variante des erfindungsgemäßen Verfahrens werden die sensorischen Neuronen im Zentrum des Ringes kultiviert und die Keratinocyten in dem äußeren Kompartiment. Die Entwicklung dieser "Co-Kultur" kann mit Hilfe von Mikroskopen untersucht werden. Die Migration oder der Durchlass von Neuriten unter der Trennscheibe oder Trennwand, bevorzugt dem Ring durch Mikrocrossing durch die Cellulose-Schmiermittelschicht konnte gefunden werden. Diese Migration gewährleistet eine Interaktion der beiden Zelltypen trotz räumlicher Trennung der Zellkörper. Das erfindungsgemäße Verfahren liefert ein Modell welches vergleichbar ist mit physiologischen Bedingungen die zwischen Keratinocyten und Nervenzellkörpern, welche in den Ganglien lokalisiert sind, vorherrschen. Unter physiologischen Bedingungen sind die Zelltypen nur durch Nervenfasern und Synapsen verbunden und damit räumlich voneinander getrennt.

Die Vorrichtung für das erfindungsgemäße Verfahren erlaubt eine getrennte Stimulierung der beiden Zelltypen in den unterschiedlichen Kompartimenten. Diese Stimulierung kann sowohl chemisch, mechanisch als auch elektronisch sein. Bei der chemischen Stimulierung nur einer Zellart muss gewährleistet sein, dass diese Substanzen nicht oder zumindest nur sehr eingeschränkt die Schicht aus Cellulose und Schmiermittel durchdringen können.

Als Schmiermittel im Sinne der Erfindung sind prinzipiell alle Mittel geeignet, die mit Cellulose eine Schicht bilden, die es ermöglicht die erfindungsgemäße Trennscheibe oder Trennwand an das erfindungsgemäße Gefäß zur Herstellung der erfindungsgemäßen Vorrichtung aus zwei Kompartimenten anzuhaften. Bevorzugt handelt es sich um Öle, Siliconöle, Wachse, Wachsester, Fette, Seifen, Salze von Fettsäuren oder Mischungen daraus, insbesondere um solche, die bei Temperaturen größer oder gleich 30 °C, bevorzugt bei den Kultivierungstemperaturen der Zellen die gewünschten Eigenschaften zeigen. Die bevorzugten Schmiermittel werden üblicherweise auch als Fette oder Öle zum Abdichten für Zentrigugen genutzt. Die vorliegende Erfindung umfaßt die Erkenntnis, dass die Schmiermittel vom Fachmann je nach gewähltem Trennwandmaterial oder je nach gewünschten Hafteigenschaften und nach gewünschtem Viskositätsgrad der Cellulose-Schmiermittelschicht gewählt werden können. Das Verhältnis der Gewichtsanteile der Einsatzmenge an Schmiermittel im Verhältnis zum Gewichtsanteil an eingesetzter Cellulose kann von 10/90 bis zu 90/10 betragen. Bevorzugt ist ein Verhältnis von 30 Anteilen Schmiermittel zu 70 Anteilen Cellulose, besonders bevorzugt ist ein Verhältnis von 50 Anteilen Schmiermittel zu 50 Anteilen Cellulose.

Im Sinne der Erfindung versteht man unter "lösliche Faktoren" alle Substanzen,die sich unter den Kultivierungsbedingungn der erfindungsgemäßen Vorrichtung in dem Kulturmedium lösen und in der Lage sind, die Membran oder die Schicht aus Cellulose und Schmiermittel, die zur Haftung der Trennscheibe oder Trennwand dient, zu durchdringen und so in das benachbarte Kopartiment zu gelangen. Bei diesen Faktoren kann es sich um Substanzen handeln, die ausgewählt sind aus der Gruppe, die gebildet wird von Wachstumsfaktoren die von den Keratinocyten gebildet werden, insbesondere der Nerven-Wachstumsfaktor NGF; Neurotransmittern; Wirkstoffe, die eine Innervierung der Zellen hervorrufen, Ionen wie beispielsweise Natrium-, Kalium- Calcium- und/oder Chloridionen.

Die Neurotransmitter können nach ihrer chemischen Beschaffenheit unterschieden werden in Aminosäuren [z. B. Glycin, L-Glutaminsäure, 4-Aminobuttersäure (GABA)], Aminosäure-Derivate (z. B. Acetylcholin), Monoamine (meist Catecholamine wie z. B. L-Noradrenalin, L-Adrenalin, Dopamin, aber auch Serotonin, Adenosin, Adenosin-5'-di- u. triphosphat), Peptide (Neuropeptide, z. B. Bradykinin, Enkephaline, Endorphine, Substanz P) und gasförmige Oxide (Stickstoffmonoxid, Kohlenstoffoxid).

In besonderen Ausführungsformen der Erfindung handelt es sich bei den Keratinocyten und Neuronen aus Spinalganglien um tierische Zellen oder es handelt sich bei den zu kultivierenden Keratinocyten und Neuronen aus Spinalganglien um menschliche Zelllinien.

In weiteren besonderen Ausführungsformen handelt es sich bei den tierischen Neuronen um Neuronen aus neugeborenen Ratten, welche ihre natürliche Reifung bereits erreicht haben. Dieses kann als eine ergänzende Bedingung für eine gute Reproduzierbarkeit der Ergebnisse im Gegensatz zur Verwendung von embryonalen Neuronen angesehen werden.

Bei den Zelllinien menschlicher Neuronen handelt es sich bevorzugt um die Zelllinie HD10.6.

In einer Ausführungsform der Erfindung wird das erfindungsgemäße Verfahren eingesetzt zur Charakterisierung freigesetzter Substanzen und/oder intervenierender Rezeptoren aus den beiden Zelltypen, insbesondere von Neuromediatoren und/oder Cytokinen.

Bei den zu charakterisierenden Substanzen und/oder Rezeptoren kann es sich sowohl um lösliche Faktoren im Sinne der Erfindung handeln oder um Substanzen und/oder Rezeptoren, die von den durch die Schicht aus Cellulose und Schmiermittel penetrierten Nervenenden der Neuronen im Kompartiment der Keratinocyten über die Nervenenden freigesetzt werden. Diese Substanzen und /oder Rezeptoren können als Neuromediatoren zu einer Innervierung der Keratinocyten führen und den Metabolismus der Keratinocyten anregen. Die Stimulierung des Metabolismus der Keratinocyten kann dann zu einer vermehrten Ausschüttung von Substanzen führen, insbesondere von Cytokinen.

In einer besondere Ausführungsform der Erfindung werden pathogene Keratinocyten verwendet um Untersuchungen durchzuführen, die ausgewählt sind aus der Gruppe, die gebildet wird von: Untersuchung pathogener Mediatoren die verantwortlich sind für kutane Krankheiten, Untersuchung der Wirkmechanismen von Wirkstoffen die zur Behandlung kutaner Krankheiten verwendet werden, Identifizierung neuer Wirkstoffe sowie Identifizierung und Optimierung neuer Behandlungsmethoden kutaner Krankheiten, insbesondere Psoriasis.

Als pathogene Mediatoren im Sinne der Erfindung werden Mediatoren verstanden, die Krankheiten erregen oder verursachen können, wie beispielsweise chemische Stoffe, Viren, Milben, Mikroorganismen wie Bakterien oder Pilze. Im Sinne der Erfindung führen diese pathogenen Mediatoren zu Krankheiten der Haut, da die Keratinocyten angegriffen und geschädigt werden.

Zu den kutanen Krankheiten deren Mediatoren mit dem erfindungsgemäßen Verfahren untersucht werden können und für die neue Wirkstoffe und deren Wirkmechanismen identifiziert werden können um neue Behandlungsmethoden zu optimieren, zählen prinzipiell alle bekannten und zur Zeit noch nicht erforschten Hautkrankheiten. Bevorzugt werden Ekzeme, Skabies oder Krätze, chronische Hautkrankheiten wie Nesselfieber und Psoriasis untersucht und insbesondere Psoriasis. Bei Psoriasis handelt es sich um eine chronische Hautkrankheit mit Bildung scharf begrenzter, gelegentlich juckender roter Herde von unterschiedlicher Größe und Gestalt, die mit silberweißen Schuppen bedeckt sind. Die Ursache der Krankheit liegt in einer Störung des Stoffwechsels der oberen Hautschichten mit beschleunigter Bildung von Zellen der Epidermis. Auslöser einer Psoriasis-Manifestation können z. B. Infektionen und Verletzungen sein.

In einer weiteren Ausführungsform wird das erfindungsgemäße Verfahren zur Untersuchung der Wirkmechanismen von Wirkstoffen an humanen Zelllinien für kosmetische Mittel verwendet, bevorzugt kosmetische Mittel für Haut, insbesondere die Kopfhaut.

In einer weiteren Ausführungsform wird das erfindungsgemäße Verfahren zur Identifizierung und Optimierung von Behandlungsmethoden für sensitive Haut verwendet.

In einer weiteren Ausführungsform wird das erfindungsgemäße Verfahren zur Untersuchung der Entwicklung von Interaktionen zwischen Keratinocyten und Neuronen, insbesondere zur Untersuchung der Bildung der Neuriten und/oder Dendriten im Kompartiment der Keratinocyten verwendet.

Eine Möglichkeit der Untersuchung der Entwicklung von Interaktionen zwischen Keratinocyten und Neuronen ist die Beobachtung und Charakterisierung der Interaktionen mit Hilfe eines Mikroskopes. An der Grenze zur Trennscheibe oder Trennwand im Kompartiment der Keratinocyten kann unter anderem beobachtet werden, ob Nervenfasern die Membran oder die Schicht aus Cellulose und Schmiermittel penetriert haben.

Weitere Techniken, die zur Charakterisierung der Interaktion als Ausführungsformen des erfindungsgemäßen Verfahrens verwendet werden, sind Techniken wie Elektrophysiologie, Lumineszenz und/oder Fluoreszenz, insbesondere Immunofluoreszenz.

Zur Identifizierung der Zellen können Immunocytochemische Verfahren verwendet werden, bei dem unterschiedliche Antikörper eingesetzt werden. Für Neuronen können beispielsweise anti-PGP 9,5 als Antikörper verwendet werden, welche die Verzweigung der Nervenfasern markieren. Es können weiterhin anti-synaptotagmine eingesetzt werden, welche vesiculare Proteine markieren, die eine entscheidende Rolle bei der Exocytose synaptischer Vesikel einnehmen, oder z.B. anti-peripherine, welche Filamente des Cytoskellets markieren. Antikörper gegen Neuropeptide wie beispielsweise Substanz P oder CGRP (Calcitonin Gene-Realted Peptide) können ebenfalls verwendet werden.

Zur Identifizierung der Keratinocyten können beispielsweise spezifische Antikörper gegen Keratin 14 verwendet werden.

In einer weiteren Ausführungsform wird das erfindungsgemäße Verfahren verwendet zur Untersuchung von Interaktionen zwischen Keratinocyten und Neuronen die ausgelöst werden durch Effekte, die ausgewählt sind aus der Gruppe, die gebildet wird von Hautalterungseffekten, Effekte durch Stressfaktoren, Effekte durch den Einfluss chemischer Substanzen und Effekte durch mechanische oder elektrische Stimulierung von Neuronen und/oder Keratinocyten.

Im Sinne der Erfindung können Hautalterungseffekte beispielsweise natürliche Hautalterungsprozesse sein, bei denen der eingeschränkte Metabolismus der Zellen zu einer verminderten Synthese von Substanzen führt, die für den Aufbau der Haut entscheidend sind. Weiterhin können oxidativ bedingte Hautalterungsvorgänge, durch radikalische Reaktionen bedingte Hautalterungsvorgänge sowie bakteriell wie hormonell bedingte Hautveränderungen, z. B. Akne darunter verstanden werden.

Ein weiterer Gegenstand der Erfindung ist ein Verfahren zur Untersuchung von Effekten durch Stressfaktoren auf die Bildung von multigeschichteten Epithelien und insbesondere auf die Bildung der Hornschicht der Haut durch Verwendung von Keratinocyten auf einem Kollagengel enthaltend Zellen ausgewählt aus der Gruppe, die gebildet wird von Fibroblasten, Neuronen, Glia-Zellen, Melanocyten und Langerhanszellen.

Im Sinne der Erfindung werden unter Stressfaktoren prinzipiell alle Faktoren bezeichnet, die einen Einfluß auf die Interaktion zwischen Keratinocyten und Neuronen ausüben, bevorzugt handelt es sich um Stressfaktoren, die den Metabolismus der Zelltypen stimulieren und insbesondere handelt es sich um Stressfaktoren, die ausgewählt sind aus der Gruppe, die gebildet wird von UV-Strahlung, Hitzeschock, osmotischer Schock und Umweltverschmutzungen, wie beispielsweise Schwermetallbelastung, Ozon oder ähnliches.

Ein weiterer Gegenstand der Erfindung ist ein Verfahren zur Untersuchung von Effekten durch chemische Substanzen auf die Bildung von multigeschichteten Epithelien und insbesondere auf die Bildung der Hornschicht der Haut durch Verwendung von Keratinocyten auf einem Kollagengel enthaltend Zellen ausgewählt aus der Gruppe, die gebildet wird von Fibroblasten, Neuronen, Glia-Zellen, Melanocyten und Langerhanszellen.

In besonderen Ausführungsformen der weiteren Gegenstände der Erfindung handelt es sich bei den Zellen um tierische Zellen oder um menschliche Zelllinien, insbesondere um die Zellen bzw Zelllinien, die auch für den ersten Gegenstand der Erfindung bevorzugt sind. Desweiteren werden durch die Verfahren der weiteren Gegenstände der Erfindung bevorzugt Wirkmechanismen von Wirkstoffen für kosmetische und/oder pharmazeutische Mittel für die Haut, insbesondere die Kopfhaut untersucht.

Ein weiterer Gegenstand der Erfindung ist eine Vorrichtung zur Bestimmung von Interaktionen zwischen Keratinocyten und Neuronen aus Spinalganglien gemäß den oben beschriebenen Interaktionen, bei der die beiden Zelltypen in Co-Kultur in zwei separaten Kompartimenten kultiviert werden, welche den Durchlass und den Austausch von Nervenfasern und/oder löslichen Faktoren zulassen.

Die mögliche Kultivierung in zwei Kompartimenten kann erfindungsgemäß errreicht werden durch eine Vorrichtung, bei der durch eine Trennscheibe oder Trennwand ein Gefäß in zwei Kompartimente geteilt wird. Bevorzugt wird die Trennscheibe oder Trennwand mit einer Mischung aus Cellulose und Schmiermitteln an das Gefäß angehaftet. Bei dem Gefäß handelt es sich bevorzugt um eine Petrischale. Bei der Trennscheibe oder Trennwand kann es sich um eine Membran, Platte, Scheibe, Wand oder einen Ring handeln, bevorzugt handelt es sich um einen Ring. Das Material dieser Trennscheibe oder der Trennwand ist im Prinzip frei wählbar zwischen Materialien, die sich durch die Kultivierungsbedingungen der beiden Zelltypen und/oder dem Haftungsmaterial nicht auflösen und nicht zerstört werden. Bevorzugt verwendet man als Material für die Trennscheibe oder Trennwand Glas.

In einer bevorzugten Variante der erfindungsgemäßen Vorrichtung werden die sensorischen Neuronen im Zentrum des Ringes kultiviert und die Keratinocyten in dem äußeren Kompartiment Die Entwicklung dieser "Co-Kultur" kann mit Hilfe von Mikroskopen untersucht werden. Die Migration oder der Durchlass von Neuriten unter der Trennscheibe oder Trennwand, bevorzugt dem Ring durch Mikrocrossing durch die Cellulose-Schmiermittelschicht konnte gefunden werden. Diese Migration gewährleistet eine Interaktion der beiden Zelltypen trotz räumlicher Trennung der Zellkörper. Das erfindungsgemäße Vorrichtung liefert ein Modell welches vergleichbar ist mit physiologischen Bedingungen die zwischen Keratinocyten und Nervenzellkörpern, welche in den Ganglien lokalisiert sind, vorherrschen. Unter physiologischen Bedingungen sind die Zelltypen nur durch Nervenfasern und Synapsen verbunden und damit räumlich voneinander getrennt.

Die erfindungsgemäße Vorrichtung erlaubt eine getrennte Stimulierung der beiden Zelltypen in den unterschiedlichen Kompartimenten. Diese Stimulierung kann sowohl chemisch, mechanisch als auch elektronisch sein. Bei der chemischen Stimulierung nur einer Zellart muss gewährleistet sein, dass diese Substanzen nicht oder nur sehr eingeschränkt die Schicht aus Cellulose und Schmiermittel durchdringen können.

Als Schmiermittel im Sinne der Erfindung sind prinzipiell alle Mittel geeignet, die mit Cellulose eine Schicht bilden, die es ermöglicht die erfindungsgemäße Trennscheibe oder Trennwand an das erfindungsgemäße Gefäß zur Herstellung der erfindungsgemäßen Vorrichtung aus zwei Kompartimenten anzuhaften. Bevorzugt handelt es sich um Öle, Siliconöle, Wachse, Wachsester, Fette, Seifen, Salze von Fettsäuren oder Mischungen daraus, insbesondere um solche, die bei Temperaturen größer oder gleich 30 °C, bevorzugt bei den Kultivierungstemperaturen der Zellen die gewünschten Eigenschaften zeigen. Die bevorzugten Schmiermittel werden üblicherweise auch als Fette oder Öle zum Abdichten für Zentrigugen genutzt. Die vorliegende Erfindung umfaßt die Erkenntnis, dass die Schmiermittel vom Fachmann je nach gewähltem Trennwandmaterial oder je nach gewünschten Hafteigenschaften und nach gewünschtem Viskositätsgrad der Cellulose-Schmiermittelschicht gewählt werden können. Das Verhältnis der Gewichtsanteile der Einsatzmenge an Schmiermittel im Verhältnis zum Gewichtsanteil an eingesetzter Cellulose kann von 10/90 bis zu 90/10 betragen. Bevorzugt ist ein Verhältnis von 30 Anteilen Schmiermittel zu 70 Anteilen Cellulose, besonders bevorzugt ist ein Verhältnis von 50 Anteilen Schmiermittel zu 50 Anteilen Cellulose.

Ein weiterer Gegenstand der Erfindung ist die Verwendung von Keratinocyten und/oder Neuronen aus Spinalganglien in Co-Kultur zur Untersuchung der Interaktion zwischen diesen Zellen, insbesondere zur Untersuchung von oben beschriebenen Effekten auf die Interaktion dieser Zellen. In einer besonderen Ausführungsform des weiteren Gegenstandes der Erfindung handelt es sich bei den Zellen um tierische Zellen oder um menschliche Zelllinien, insbesondere um die Zellen bzw Zelllinien, die auch für den ersten Gegenstand der Erfindung bevorzugt sind.

Die erfindungsgemäßen Verfahren, beorzugt der erste Gegenstand der Erfindung sowie die erfindungsgemäße Vorrichtung und die erfindungsgemäßen Verwendungen ermöglichen die detailierten Untersuchungen der Interaktionen und gegenseitigen Signalisierung zwischen sensorischen Neuronen und Keratinocyten. Unter anderem werden folgende Untersuchungen möglich:
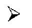 die Effekte von Neuron-Keratinocyten Interaktionen,
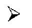 die Rolle und der natürliche Charakter des Neuronen-Keratinocyten Kontaktes in der Entwicklung dieser zwei Zelltypen,
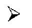 die Effekte der Innervierung der mitotischen Aktivität der Keratinocyten,
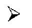 die Bestimmung der intrazellulären Konzentration an Calcium-lonen in Keratinocyten nach der elektrischen Stimulierung der Neuronen,
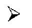 die Bestimmung der intrazellulären Konzentration an Calcium-lonen in Neuronen nach einer mechanischen oder chemischen Stimulierung der Keratinocyten,
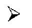 die Charakterisierung freigesetzter Substanzen und/oder Rezeptoren aus den beiden Zelltypen, insbesondere von Neuromediatoren und/oder Cytokinen,
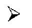 die Konsequenzen von Entzündungen, externen Stimulierungen, UV-Strahlung und anderen Effekten auf Neuronen und Keratinocyten und auf die Interaktion zwischen beiden Zelltypen,
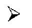 die Konsequenzen von natürlichen Hautalterungsprozessen für die Interaktionen zwischen Neuronen und Keratinocyten.
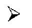 eine detailiertere Charakterisierung der Interaktionen durch Techniken wie Elektrophysiologie (patch clamp) und durch Bildanalysen
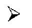 die Suche nach Wirkstoffen mit einer Aktivität auf Neuronen, Neuropeptiden und Keratinocyten für kosmetische und/oder pharmazeutische Verwendungen.
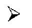 die Untersuchung kutaner Krankheiten in Verbindung mit der Interaktion zwischen Neuronen und Keratinocyten.

Für die Suche nach Wirkstoffen und für die Untersuchung kutaner Krankheiten für kosmetische Verwendungen werden in den erfindungsgemäßen Verfahren menschliche Zelllinien verwendet.

### Beispiele

### 1. Gewinnung der Neuronen aus Spinalganglien

Die Neuronen wurden erhalten aus Geweben von neugeborenen Ratten nach der Methode von Scott bzw. Dichter, beschrieben in: 1. *Scott BS, Adult mouse dorsal root ganglia neurons in cell culture, J. Neurobiol., 8: 417-427, 1977* und 2.. *Dichter MA, Fischbach GD, The action potential of chick dorsal root ganglion neurones maintained in cell culture, J. Physiol, 267: 281-298, 1977.*

Die Neuronen aus neugeborenen Ratten, die maximal eine Woche alt waren, wurden in eine Petrischale mit PBS gegeben und gekühlt. Sie wurden mit einem Skalpel gereinigt um die Verzweigung der Nervenfasern zu unterdrücken. Anschliesend wurden die Zellen zweifach mit PBS ohne Calcium-lonen und ohne Magnesium-Ionen gewaschen und einer enzymatischen Trypsin Behandlung (0,05 %) unterzogen. Nach Erhalt der Zellsuspension wurde der Überstand der Lösung 8 min bei 150 G zentrifugiert. Der Rückstand wurde gewaschen und in DMEM-Medium gegeben.

Die zelluläre Suspension enthaltend Neuronen und Glia-Zellen wurde mit einem Partikelzähler (Mallassey slide) ausgezählt. Die Suspension wurde anschliessend mit einer Dichte von 150000 Zellen/cm² kultiviert.

### 2. Gewinnung der Keratinocyten

Die Keratinocyten wurden kultiviert entsprechend der Methode von Rheinwald et al. beschrieben in: *Rheinwald* JG, Green H, *Serial cultivation of strains of human epidermal keratinocytes: the formation of keratinising colonies from single cells, Cell, 6: 331-434, 1975*.

Es wurden neugeborene Ratten, die maximal 24 Stunden alt waren, durch Kälte anesthesiert und getötet. Anschliessend wurden ca. 1 cm² große Hautstückchen ausgeschnitten und durch Trypsin (0,2 %) enzymatisch verdaut.

### 3. Kultivierung der Zellen

Die Zellen wurden kultiviert in einem Gefäß, welches vorher mit Poly-Lysin oder Collagen beschickt wurde um eine Zellhaftung zu erreichen. Keratinocyten wurden nur auf Collagen kultiviert, während die Neuronen auf beiden Medien kultiviert werden konnten. Die Kultivierung erfolgte bei 37 °C.

Zur Co-Kultivierung wurde in eine Petrischale ein Glasring mit einer Mischung aus Cellulose und Schmiermittel angeheftet und fixiert. Die Neuronen wurden in dem inneren Kompartiment und die Keratinocyten ausserhalb des Ringes im äußeren Kompartiment kultiviert.

Je nach gewünschter Untersuchung wurden die Kultivierungszeiten und Bedingungen sowie die Techniken zur Auswertung der Ergebnisse variiert.

## Patentansprüche

1. Verfahren zur Bestimmung von Interaktionen zwischen Keratinocyten und Neuronen aus Spinalganglien bei dem die beiden Zelltypen in Co-Kultur in zwei separaten Kompartimenten kultiviert werden, welche den Durchlass und den Austausch von Nervenfasern und/oder löslichen Faktoren zulassen.

2. Verfahren nach Anspruch 1, **dadurch gekennzeichnet, dass** es sich bei den Keratinocyten und Neuronen aus Spinalganglien um tierische Zellen oder um menschliche Zelllinien handelt.

3. Verfahren nach Anspruch 2, **dadurch gekennzeichnet, dass** es sich bei den tierischen Nervenzellen um Neuronen aus Spinalganglien von neugeborenen Ratten handelt.

4. Verfahren nach Anspruch 2, **dadurch gekennzeichnet, dass** es sich bei den Nervenzellen aus Spinalganglien um Zelllinien menschlicher Neuronen handelt, insbesondere um die Zelllinie HD10.6.

5. Verfahren nach einem der Ansprüche 1 bis 4 zur Charakterisierung freigesetzter Substanzen und/oder intervenierender Rezeptoren aus den beiden Zelltypen, insbesondere von Neuromediatoren und/oder Cytokinen.

6. Verfahren nach einem der Ansprüche 1 bis 4, **dadurch gekennzeichnet, dass** es sich bei den verwendeten Keratinocyten um pathogene Keratinocyten handelt und Untersuchungen durchgeführt werden, die ausgewählt sind aus der Gruppe,die gebildet wird von: Untersuchung pathogener Mediatoren die verantwortlich sind für kutane Krankheiten, Untersuchung der Wirkmechanismen von Wirkstoffen die zur Behandlung kutaner Krankheiten verwendet werden, Identifizierung neuer Wirkstoffe sowie Identifizierung und Optimierung neuer Behandlungsmethoden kutaner Krankheiten, insbesondere Psoriasis.

7. Verfahren nach einem der Ansprüche 1, 2 oder 4 zur Untersuchung der Wirkmechanismen von Wirkstoffen an humanen Zelllinien für kosmetische Mittel, bevorzugt kosmetische Mittel für Haut, insbesondere die Kopfhaut.

8. Verfahren nach einem der Ansprüche 1, 2 oder 4 zur Identifizierung und Optimierung von Behandlungsmethoden für sensitive Haut.

9. Verfahren nach einem der Ansprüche 1 bis 8 zur Untersuchung der Entwicklung von Interaktionen zwischen Keratinocyten und Neuronen, insbesondere die Untersuchung der Bildung der Neuriten und/oder Dendriten im Kompartiment der Keratinocyten.

10. Verfahren nach einem der Ansprüche 1 bis 9, **dadurch gekennzeichnet, dass** die Interaktionen zwischen Neuronen und Keratinocyten dass die Interaktionen weiterhin charakterisiert werden durch Techniken wie Elektrophysiologie, Lumineszenz und/oder Fluoreszenz, insbesondere Immunofluoreszenz.

11. Verfahren nach einem der Ansprüche 1 bis 4 zur Untersuchung von Interaktionen zwischen Keratinocyten und Neuronen die ausgelöst werden durch Effekte, die ausgewählt sind aus der Gruppe, die gebildet wird von, Hautalterungseffekten, Effekte durch Stressfaktoren, Effekte durch den Einfluss chemischer Substanzen und Effekte durch mechanische oder elektrische Stimulierung von Neuronen und/oder Keratinocyten.

12. Verfahren zur Untersuchung von Effekten durch Stressfaktoren auf die Bildung von multigeschichteten Epithelium und insbesondere auf die Bildung der Hornschicht der Haut durch Verwendung von Keratinocyten auf einem Kollagengel enthaltend Zellen ausgewählt aus der Gruppe, die gebildet wird von Fibroblasten, Neuronen, Glia-Zellen, Melanocyten und Langerhanszellen.

13. Verfahren nach Anspruch 11 und/oder 12, **dadurch gekennzeichnet, dass** die Stressfaktoren einen Einfluß auf die Interaktion zwischen Keratinocyten und Neuronen ausüben, bevorzugt handelt es sich um Stressfaktoren, die den Metabolismus der Zelltypen stimulieren und insbesondere handelt es sich um Stressfaktoren,die ausgewählt sind aus der Gruppe, die gebildet wird von UV-Strahlung, Hitzeschock, osmotischer Schock und Umweltverschmutzung.

14. Verfahren zur Untersuchung von Effekten durch chemische Substanzen auf die Bildung von multigeschichteten Epithelium und insbesondere auf die Bildung der Hornschicht der Haut durch Verwendung von Keratinocyten auf einem Kollagengel enthaltend Zellen ausgewählt aus der Gruppe, die gebildet wird von Fibroblasten, Neuronen, Glia-Zellen, Melanocyten und Langerhanszellen.

15. Verfahren nach einem der Ansprüche 12 bis 14, **dadurch gekennzeichnet, dass** es sich bei den Zellen um tierische Zellen oder um menschliche Zelllinien handelt.

16. Verfahren nach einem der Ansprüche 12 bis 15 zur Untersuchung der Wirkmechanismen von Wirkstoffen für kosmetische und/oder pharmazeutische Mittel für die Haut, insbesondere die Kopfhaut.

17. Vorrichtung zur Bestimmung von Interaktionen zwischen Keratinocyten und Neuronen aus Spinalganglien gemäß Ansprüche 1 bis 11, bei der die beiden Zelltypen in Co-Kultur in zwei separaten Kompartimenten kultiviert werden, welche den Durchlass und den Austausch von Nervenfasern und/oder löslichen Faktoren zulassen.

18. Verwendung von Keratinocyten und/oder Neuronen aus Spinalganglien in Co-Kultur zur Untersuchung der Interaktion zwischen diesen Zellen, insbesondere zur Untersuchung von Effekten gemäß Anspruch 11, 13 und 14 auf die Interaktion dieser Zellen.

19. Verwendung nach Anspruch 17, **dadurch gekennzeichnet, dass** es sich bei den Zellen um tierische Zellen oder um menschliche Zelllinien handelt.
